(19) Europäisches Patentamt / European Patent Office / Office européen des brevets

(11) **EP 4 177 902 A1**

(12) **EUROPEAN PATENT APPLICATION**

(43) Date of publication:
**10.05.2023 Bulletin 2023/19**

(21) Application number: **21206570.0**

(22) Date of filing: **04.11.2021**

(51) International Patent Classification (IPC):
**G16H 20/10** *(2018.01)* **G16H 50/50** *(2018.01)*

(52) Cooperative Patent Classification (CPC):
**G16H 50/50; G16H 20/10**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Universität Basel Vizerektorat Forschung**
**4001 Basel (CH)**
• **Universität Konstanz**
**78464 Konstanz (DE)**

(72) Inventors:
• **SZINNAI, Gabor**
**4102 Binningen (CH)**

• **KOCH, Gilbert**
**88662 Überlingen (DE)**
• **PFISTER, Marc**
**3652 Hilterfingen (CH)**
• **SCHROPP, Johannes**
**78462 Konstanz (DE)**
• **STEFFENS, Britta**
**78464 Konstanz (DE)**
• **BACHMANN, Freya**
**78467 Konstanz (DE)**

(74) Representative: **Schulz Junghans**
**Patentanwälte PartGmbB**
**Großbeerenstraße 71**
**10963 Berlin (DE)**

(54) **METHOD, SYSTEM AND COMPUTER PROGRAM FOR COMPUTATION OF OPTIMAL INDIVIDUAL DOSING REGIMEN, PARTICULARLY SUBJECT TO CLINICAL CONSTRAINTS**

(57) The present invention relates to a method for automatically determining an optimal individual dosing regimen of at least one drug for a patient suffering from a known disease, the optimal individual dosing regimen being optionally subject to at least one clinical constraint, wherein the method comprises the steps of: providing a mathematical model adapted to model a progression of said disease and an effect of the at least one drug on the progression of the disease, the model comprising individual model parameters associated with the patient; utilizing an empirical Bayesian estimation to automatically and numerically estimate the individual model parameters of the mathematical model utilizing patient data associated with the patient; automatically calculating an optimal individual dosing regimen for the mathematical model by solving an optimal control problem based on a desired progression of the disease, the estimated individual model parameters, and an initial guess for the dosing regimen; and adjusting the optimal individual dosing regimen to optionally account for at least one clinical constraint to yield the optimal individual dosing regimen subject to said at least one clinical constraint.

Fig. 1

**Description**

**Specification**

The present invention relates to a method, a system and a computer program for determining an optimal dosing regimen of at least one drug for a patient suffering from a known disease.

A majority of diseases are still difficult to treat at the individual level as disease progression differs among patients and clinical practice. Particularly, it often remains a challenge to determine the optimal individual dosing regimen, particularly in a clinical setting, in a sufficiently fast and reliable fashion that ideally allows to control the disease progression, while minimizing treatment related adverse events, resulting in recovery of the patient.

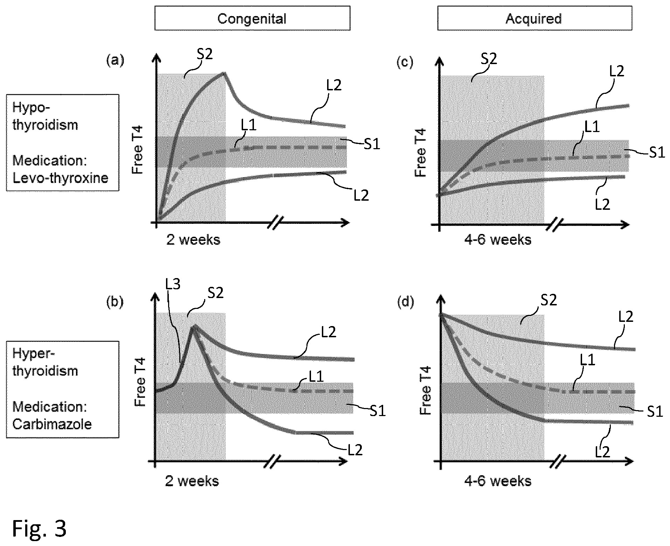

Particularly, among such diseases, congenital hypothyroidism is the most frequent congenital endocrine disorder and the most frequent preventable cause of mental retardation worldwide [Polak 2017]. Disease severity strongly varies among individuals at diagnosis, i.e. the thyroid-stimulating hormone (TSH) is in the range of 6-1200mU/L whereas the norm is 0.5-5 mU/L. Main effects of thyroid hormones are axonal outgrowth of interneurons and myelination, processes that are ongoing in humans mainly after birth. Neonatal screening allows diagnosing of children with congenital hypothyroidism, before they suffer from clinical signs and irreversible neurological deficiencies. During the last 40 years, the neurological outcome of patients with congenital hypothyroidism has been strongly improved 1) by shortening the time window between screening and onset of treatment (currently 4-5 days in Switzerland) and 2) by increasing starting doses of levothyroxine (from 5-8 mcg/kg/d to currently 10-15 mcg/kg/d). The goal of substitutive treatment with levothyroxine is to correct hypothyroidism as rapidly as possible to protect brain development. The clinician aims to reach free thyroxine (T4) levels in the upper normal reference range within 14 days (cf. Fig. 3 (a)). However, several studies revealed that up to 10% of children were overdosed with a starting and maintenance dose of 10-15 mcg/kg/d over relevant time periods. Further, at age of 11 years their neurological outcome was comparable to children with periods of underdosing during follow-up, and both over- and underdosed children had a worse neurological outcome compared to children with levothyroxine doses maintaining thyroid hormones in the reference range during follow-up.

Current international guidelines recommend more frequent controls during the first 2 years of life, to allow more frequent and necessary individual, age-appropriate dose adjustments maintaining thyroid hormones in a physiological normal reference range. Although this permits a faster detection of over- and underdosing, it does not prevent episodes of suboptimal dosing. As a consequence, the last step for improving neurological outcome in affected patients must focus on optimization of long-term substitutive doses by considering clinical and laboratory data for personalized dosing during follow-up. Therefore, it is particularly highly desirable to be able to determine optimal personalized doses during the first 2 years of life, which are essential for brain development and long-term normal cognitive function. Central congenital hypothyroidism is a subgroup of congenital hypothyroidism with low TSH and FT4 values due to disorders of the pituitary gland and the hypothalamus treated with levothyroxine in the same way.

Furthermore, congenital hyperthyroidism occurs in about 10% of offspring of mothers with autoimmune hyperthyroidism (Graves' disease) due to transplacental passage of stimulating antibodies against the thyrotropin (TSH)-receptor. It puts infants to significant risk for morbidity and mortality. In contrast to infants with congenital hypothyroidism, who are in 95% asymptomatic at birth and during the first weeks of life, infants suffering from congenital hyperthyroidism present with typical signs and symptoms, ranging from mild tachycardia and sweating to weight loss, vomiting, diarrhoea, dehydration, severe hyperthermia, seizures, and cardiac insufficiency. Onset is either directly at birth or delayed by transplacental passage of maternal anti-thyroid medication until day 4-10 of life. Again, severity of disease is very variable ranging from very mild forms to life-threatening complications. The delayed onset further complicates optimal treatment, making clinical observation and laboratory controls mandatory at day 1, 4, 7, 10 and 14 of life.

Also, for congenital hyperthyroidism optimizing the treatment by anticipating which child is at risk for severe clinical course and when treatment should be started, and by finding the minimal effective starting and maintenance dose adapted for disease severity in the context of possible severe side effects of anti-thyroid drugs (agranulocytosis, hepatitis, vasculitis) is highly desirable (cf. Fig. 3 (b)).

Furthermore, acquired hyperthyroidism [Leger 2014] occurs in 0.5-2:100000 in children in Europe and in 25-90:100000 in adults in Europe, and has highest prevalence in Brazil, China and India ranging from 0.75-1.25% of the population, respectively. Severity of hyperthyroidism again strongly varies among individuals at diagnosis, with FT4 values ranging from 22 to >100 pmol/L whereas the norm is 11-21 pmol/L. Restoration of physiological FT4 levels should be achieved after 4-6 weeks, in contrast to congenital thyroid disease, where normalization should be aimed as rapidly as possible. A simple treatment approach was in former years a block-and-replace strategy: The dose of anti-thyroid drugs was chosen to completely block the thyroid, and substitution with levothyroxine was added. According to international guidelines, in the context of increasing frequency of side effects with higher doses of anti-thyroid drugs (agranulocytosis, hepatitis, vasculitis), such an approach is not recommended anymore according to international guidelines. Currently, the minimal effective dose of the anti-thyroid agent has to be titrated on an individual level to mitigate the risk of overdosing causing iatrogenic hypothyroidism or underdosing resulting in persistent hyperthyroidism. Both hyper- and hypothyroidism have negative effects on growth and pubertal development (retardation or acceleration, respectively), and on cognitive function leading to a decrease in school and sport performance. Finally, to further increase complexity during childhood, the disease course is more severe and risk of hyperthyroidism recurrence is much higher in young or prepubertal children compared to older (>10 years) or pubertal and postpubertal children, rendering childhood Graves' disease a therapeutic challenge.

Also for Graves' disease, treatment strategies that allow one to compute the optimal individual starting dose to reach steady-state and minimal effective maintenance dose to avoid recurrence during follow-up depending on clinical and laboratory parameters are highly desirable (cf. Fig. 3 (d)).

Furthermore, hypothyroidism is common throughout the world in an adult population with a prevalence ranging from 0.2-5.3% in Europe. The most frequent cause of acquired hypothyroidism is Hashimoto thyroiditis in iodine sufficient areas of the world. The aim for treatment of acquired hypothyroidism (incidence rate of 1.2%) is to normalize free T4 levels slowly, to avoid signs and symptoms of hyperthyroidism after longstanding hypothyroidism. In analogy with the other diseases, an optimal starting and maintenance dose is necessary to normalize somatic growth, cognitive function and consequently school performance but not overdose the pediatric patient (cf. Fig. 3 (c)). Further, central hypothyroidism due to disorders of the pituitary gland and the hypothalamus (e.g. trauma, tumor, infection, a.o.) may cause a further form of acquired hypothyroidism.

In summary, based on the above, the problem to be solved by the present invention is to provide a method, a system, a computer program, a computer-readable non-transitory storage medium, and a data carrier signal that allow to improve personalized dose finding, particularly for pediatric thyroid diseases. Due to the severity of such diseases it is of high clinical relevance to improve treatment and outcome of affected patients.

This problem is solved by a method having the features of claim 1, a system having the features of claim 11, a computer program having the features of claim 13, a computer-readable non-transitory storage medium having the features of claim 14, and by a data carrier signal having the features of claim 15.

Preferred embodiments of these aspects of the present invention are stated in the corresponding dependent claims and are described below.

According to claim 1, a method for automatically determining an optimal individual dosing regimen of at least one drug for a patient suffering from a disease, wherein particularly the optimal individual dosing regimen is subject to at least one clinical constraint, wherein the method comprises the steps of:

(a) Providing a mathematical model adapted to model a disease progression of said disease and an effect of the at least one drug on the progression of the disease, the model comprising individual model parameters associated with the patient,

(b) Using an empirical Bayesian estimation to automatically and numerically estimate the individual model parameters of the mathematical model using patient data,

(c) Automatically calculating an optimal individual dosing regimen for the mathematical model by solving an optimal control problem based on a desired disease progression, the estimated individual model parameters, and an initial guess for the dosing regimen,

Advantageously, the present invention allows a personalized dosing so as to raise medical therapy to a next level. As for many diseases no new pharmacologic approaches are under development, particularly not for hyper- or hypothyroidism, improving on determining individual dosing regimen is the key in improving therapy outcomes.

Particularly, as outlined above and indicated in Figure 3, the present invention is particularly applicable to modeling the different clinical situations that require to be modeled separately to develop disease specific algorithms for starting and maintenance dosages.

In the framework of the present invention, the following notions and definitions are frequently referred to.

Patient

A patient can be a virtual / in-silico (non-human) object (e.g., the average of a patient population) or a real human patient.

Visit

A visit of a patient can be (i) any time point of interest, (ii) the time point of a clinical visit of a non-hospitalized patient at the hospital, (iii) or the time point at which the clinician sees a hospitalized patient in the hospital.

### Wearable devices and sensors

Wearable devices and sensors are noninvasive devices that are worn by the patient and monitor vital signs such as heart rate (beats per minute), blood pressure (mmHg), body temperature (°C), respiratory rate (frequency/minute), oxygen saturation and steps (counts per day).

### Laboratory measurements

Laboratory measurements are routine biochemical tests of biomarkers (e.g., hormones) analyzed in medical laboratories or by health care providers like physicians, nurses, medical assistants or pharmacists. In case of thyroid hormones, one hormone is synthesized and secreted by the pituitary gland (regulatory hormone of the peripheral endocrine gland called thyrotropin or thyroid-stimulating-hormone (TSH) for the pituitary thyroid axis) and peripheral hormones are secreted from the thyroid (endocrine gland under control of the pituitary gland which are thyroxine (T4) and triiodothyronine (T3) in bound (T3, T4) and free forms (FT3, FT4)).

### Demographic / anthropometric characteristics

Demographic data are, e.g., age (days (neonate), months (infant), years (child or adult)), gestational age at birth (premature or term born) and gender. Anthropometric data are, e.g., body weight (kg), height (cm) and head circumference (cm).

### Covariates

A covariate is a patient characteristic (e.g., a demographic / anthropometric characteristic) that can, but not necessarily must, vary over time. A time-invariant covariate is e.g., birth weight, gestational age, delivery mode, all characteristics at baseline etc. Examples for a time-varying covariate are weight, height, heart rate, blood pressure, respiratory rate etc.

### Patient data

The patient data is the combination of data from a wearable device or sensor, laboratory measurements, and covariates from all available visits. In addition, the patient data contains all information about administered doses from all available visits.

### Pharmacokinetic model

The pharmacokinetic (PK) model describes the concentration of a drug (or e.g., its metabolite) or an exogenous substance, by e.g., absorption, distribution, metabolism and elimination processes. Route of administrations of the drug can be (i) oral, (ii) intravenous, or (iii) subcutaneous. A PK model is typically characterized by one or more differential equations.

### Pharmacokinetic / Pharmacodynamic model

The pharmacokinetic / pharmacodynamic (PKPD) model describes the response of a biomarker or a disease progression etc. (the so-called pharmacodynamic) caused by stimulatory / inhibitory effects of one or several drugs e.g., characterized by a PK model. For a combination of several drugs, the drugs can be administered simultaneously or sequentially. A PKPD model is typically characterized by one or more differential equations.

### Basic Mathematical model

The basic mathematical model can be a PK model only, or a PKPD model. The basic mathematical model may include a combination of physiologically based mechanistic assumptions, maturation related processes and/or data-driven assumptions. The basic mathematical model is controlled by the administered doses of one or several drugs. The basic mathematical model contains model parameters summarized in the vector $\theta$, some of them might be already known while others need to be estimated. The disease state is one state variable or a combination of several state variables of the basic mathematical model that characterizes the disease progression.

Individual model parameters

The individual model parameters are summarized in the vector $\theta_i$ and characterize an individual patient based on the individual data. Index i denotes the i-th individual in a population with *N* patients, i.e., *i* = 1, ..., *N*. All or just some model parameters can have physiological, biological or other meanings. For example, a model parameter can describe the rate of a process (e.g., production or elimination), can describe the volume of a compartment, or can have other meanings.

Desired disease progression

The desired disease progression is given by the clinician / user and characterizes the goal for the individual patient. For example, for a patient showing an increased biomarker that is treated with a drug, the goal is to normalize the biomarker value to the normal (age-specific) reference range within a given time period. Or, in the example of a PK model, the desired disease progression can be a goal regarding drug-exposure, e.g., achieving a certain area under the concentration curve. The desired disease progression under pharmacological therapy is characterized by a mathematical function.

Dosing regimen

The dosing regimen is the information about the administration of one drug or several drugs. It contains the drug(s) itself, the route of administration (oral, intravenous, subcutaneous), the dosing time points, and the doses.

Optimal individual dosing regimen

The optimal individual dosing regimen is the dosing regimen with the optimal (with respect to the desired disease progression) doses for an individual patient.

Optimal individual dosing regimen subject to constraints in clinical application

The optimal individual dosing regimen subject to constraints in clinical application is the optimal individual dosing regimen adjusted to constraints in clinical application. This can be, e.g., available tablet sizes. Different strategies can be applied to adjust an optimal dose between two available dosing sizes, e.g.,

1) Round to the closest available dosing size, or
2) Choose upper or lower available dose based on the lower corresponding cost functional value (as in mixed-integer optimization [Belotti 2013])

Non-linear mixed effects modeling approach

In the non-linear mixed effects (NLME) modeling approach [Lavielle 2014] it is assumed that patients with the same disease form a population having common or similar features. For each individual *i*, the vector of $n_i$ measurements $w_i = (w_{i1}, ..., w_{in_i})$ at time points $t_i = (t_{i1}, ..., t_{in_i})$ are taken for *i* = 1, ..., *N*. The prediction *f* from the basic mathematical model forecast these measurements. However, the individual model parameters $\theta_i$ in the basic mathematical model are unknown or at least partially unknown. A subset of these model parameters are the estimated individual model parameters $\phi_i$. The basic NLME modeling approach reads

$$\phi_i = \phi_{pop} + \beta c_i + \eta_i$$

$$w_{ij} = f(t_{ij}, \phi_i) + \epsilon_{ij}$$

for *i* = 1, ..., *N* and *j* = 1, ..., $n_i$ where $\eta_i$ are the random effects with $\eta_i \sim \mathcal{N}(0, \Omega)$ and covariance matrix $\Omega$, $\beta$ denotes the covariate effects, $c_i$ the individual covariate values, and $\phi_{pop}$ the parameters of the typical (average) individual in the population. The individual model parameters $\theta_i$ utilized in the basic mathematical model contain the estimated individual model parameters $\phi_i$ as well as known parameters, and $\varepsilon_i$ is the residual error for which different error models can be considered. Hence, the individual model parameters $\phi_i$ are treated as random variables and are assumed to be independently and normally distributed. Other distributions can be realized by transformation $\phi = h(\psi)$, e.g., $h(x) = \log(x)$ for log-normally distributed model parameters $\psi$. The population parameters $\rho = (\phi_{pop}, \Omega, \beta)$ are estimated by maximizing the so-called observed likelihood

$$p((w_1, \ldots, w_N); \rho) = \prod_{i=1}^{N} \int p(w_i|\phi_i; \rho)\, p(\phi_i; \rho)\, d\phi_i$$

(equivalently the log-likelihood) describing the probability to achieve the observed measurements $w_1, \ldots, w_N$ with respect to $\rho$, i.e., the $\rho$ is chosen such that the model most likely predicts the observed measurements. The observed likelihood depends on the conditional probability density functions $p(w_i|\phi_i; \rho)$ and the marginal probability density functions $p(\phi_i; \rho)$.

Mathematical model

The mathematical model is a basic mathematical model together with the application of the NLME modeling approach to describe a given population of patients by its population parameters $\rho$. Such a mathematical model is therefore sometimes also denoted as fully developed mathematical model.

Empirical Bayesian Estimation

The empirical Bayesian estimation also known as maximum a posteriori estimation [Bassett, Deride 2019] computes the estimated individual model parameters $\phi_i$ which most likely predict the observed measurements $w_i$ at time points $t_i$ for the individual i belonging to a population with population parameters $\rho$. Mathematically, the conditional probability density function $p(\phi_i|w_i; \rho)$ is maximized or equivalently, minus twice the log-likelihood is minimized, i.e.,

$$\phi_i = \mathrm{argmin}\left\{-2\log p(\phi_i|w_i; \rho)\right\}$$

Optimal Control Problem

An optimal control problem is solved to compute a control (i.e., the doses of one or several drugs) such that one state variable or a combination of several state variables of the mathematical model (e.g., the disease state of the patient) reaches the desired disease progression as closely as possible. This is achieved by minimizing a cost functional which characterizes the difference between the disease state resulting from a particular control and the desired disease progression. The optimal control problem can be solved with an optimal control algorithm such as the OptiDose algorithm disclosed in the article: "Bachmann F, Koch G, Pfister M, Szinnai G, Schropp J (2021) OptiDose: Computing the Individualized Optimal Drug Dosing Regimen Using Optimal Control. Journal of Optimization Theory and Applications 189:46-65", which is hereby incorporated herein by reference in its entirety. Particularly, the optimization is an iterative procedure (i) starting with an initial control, (ii) solving the mathematical model to obtain the disease state, (iii) solving the adjoint equation (linking disease state and cost functional) and (iv) updating the control utilizing the gradient of the cost functional. Steps (ii)-(iv) are repeated in each iteration.

According to a preferred embodiment of the method according to the present invention, the method further comprises the step of:

(d) Adjusting the optimal individual dosing regimen to account for at least one clinical constraint to yield the optimal individual dosing regimen subject to said at least one clinical constraint.

Further, according to an embodiment of the method, the mathematical model is or comprises a pharmacokinetic-pharmacodynamic (PKPD) model.

Further, according to an embodiment of the method, the desired disease progression is given in form of a mathematical function.

Further, according to an embodiment of the method, steps (b) to (c), particularly (b) to (d), are conducted at each visit of a plurality of x succeeding visits of the patient, wherein for $x > 1$ the patient data in step (b) includes all patient data of the previous $x - 1$ visit(s) and the empirical Bayesian estimation in step (b) further uses all doses of said at least one drug actually administered to the patient until the current visit.

Further, according to an embodiment of the method, at least a part of an algorithm performing steps (b) to (c), particularly (b) to (d), is approximated by an artificial neural network (ANN).

Artificial neural networks (ANN) are universal approximators and can therefore approximate any input/output relationship up to a certain accuracy depending on the amount of available training data and the structure of the ANN. Hence, an ANN can also approximate any algorithm up to a certain accuracy, i.e., it can learn the functionality of the algorithm and finally mimic the algorithm. The advantage of a trained ANN is that it can compute the learned input/output relationship in a split second on standard computers and hence can replace a computationally time-consuming algorithm.

Particularly, according to a preferred embodiment, the steps (a) to (c), and optionally step (d) are approximated by an ANN.

Particularly, for this, in an embodiment, multiple simulations with the respective part of the algorithm / step of the method that shall be substituted/approximated by the ANN are conducted, wherein these simulations are then used as training data for the ANN, and wherein resulting trained ANN will be included in the iterative algorithm (or used in the method) instead. Hence, the computational cost of some parts of the algorithm can be outsourced into the algorithm development by training an ANN upfront. This can significantly reduce the computational cost for daily clinical application.

Furthermore, according to an embodiment of the method, the disease is one of: acquired hypothyroidism (autoimmune and non-autoimmune forms), acquired hyperthyroidism (autoimmune and non-autoimmune forms), congenital hypothyroidism, congenital hyperthyroidism.

Furthermore, according to an embodiment of the method, the drug is selected from the group comprised of: levothyroxine ($C_{15}H_{11}I_4NO_4$, CAS number 51-48-9) for treatment of any form of hypothyroidism, carbimazole ($C_7H_{10}N_2O_2S$, CAS number 22232-54-8), methimazole ($C_4H_6N_2S$, CAS number 60-56-0), propylthiouracil ($C_7H_{10}N_2OS$, CAS number 51-52-5) for treatment of any form of hyperthyroidism.

Furthermore, according to an embodiment of the method, at least a portion of said patient data is measured by a wearable device worn by the patient.

Wherein the wearable device is one of: a watch, particularly a smart watch, a mobile phone, particularly a smart phone. Particularly, a smart phone is a mobile phone comprising at least one processor configured to execute computer programs and a display for displaying information, wherein the display can be a touch screen forming part of a user interface. Likewise, a smart watch can in particular be a watch comprising at least one processor configured to execute computer programs and a display for displaying information, wherein the display can be a touch screen forming part of a user interface of the watch.

Further, according to an embodiment of the method according to the present invention, said patient data comprises a vital sign such as the heart rate of the patient, wherein the vital sign (e.g. heart rate) of the patient is measured with the wearable device. Other vital signs/physiological data that can be measured by the wearable device are at least one of: blood pressure (mmHg), body temperature (°C), respiratory rate (frequency/minute), oxygen saturation, steps (counts per day).

Yet another aspect of the present invention relates to a system for automatically determining an optimal individual dosing regimen of at least one drug for a patient suffering from a known disease (the optimal individual dosing regimen being optionally subject to at least one clinical constraint), the system comprising at least one processor configured to perform the steps of the method according to the present invention.

According to an embodiment of the system, the system further comprises a wearable device configured to measure at least a portion of said patient data. Particularly, the wearable device can be configured to measure physiological data of the patient. In an embodiment the wearable device can be configured to measure a heart rate.

A computer program comprising instructions, which when carried out on a computer or on the system according to the present invention, cause the computer (or system) to carry out the steps of the method according to the present invention. Particularly, the system or computer is caused to receive patient data, particularly a heart rate measured by the wearable device (or another vital sign described herein), particularly in order to use said patient data in the method according to the present invention as described herein.

A further aspect of the present invention relates to a computer-readable non-transitory storage medium having stored thereon the computer program according to the present invention.

Furthermore, yet another aspect of the present invention relates to a data carrier signal carrying the computer program according to the present invention.

In the following, further features and advantages of the present invention as well as embodiments of the present invention shall be described with reference to the Figures, wherein

Fig. 1    displays an overview of all components according to an embodiment of the invention and the relationships between all components. (1)-(4) are the four components of an embodiment of the method / algorithm according to the present invention. (A)-(G) are the additional components that interact with components (1)-(4),

Fig. 2    schematically illustrates an iterative procedure over the number of visits carried out by an embodiment of the method / algorithm according to the present invention, wherein x denotes the current visit,

Fig. 3    shows a schematic overview of the four thyroid disease situations as an example of an application of the present invention. Normal range of thyroid hormones (free thyroxine (FT4)) is marked by the horizontal shaded area S1 and the desired time interval for normalization of FT4 values in all four thyroid diseases is highlighted by another shaded area. In congenital hyper- and hypothyroidism, FT4 should be at the upper normal reference range as rapidly as possible to protect brain development, ideally within 2 weeks. In acquired hyper- and hypothyroidism FT4 values should not increase or decrease too rapidly to avoid side effects of therapy, ideally the normal reference range is reached within 4-6 weeks. Desired FT4 increase or decline is depicted by the dashed line L1 in each graph. Over- or underdosing is depicted by solid lines L2 above and below the normal range (horizontal shaded area S1). Medication for congenital and acquired hypothyroidism is levothyroxine, and medication for congenital and acquired hyperthyroidism is carbimazole, methimazole or propylthiouracil. a) congenital hypothyroidism, b) congenital hyperthyroidism, the solid line L3 depicts the most frequent delayed peak of hyperthyroidism between day 4 and 10 of life, c) acquired hypothyroidism, d) acquired hyperthyroidism. Interrupted time axis depicts initial correction period of hyper- and hypothyroidism and long-term maintenance period of treatment, and

Fig. 4    shows an example of the optimal individual dosing regimen computed by the method/computer program according to the present invention.

Components of an embodiment of the method/algorithm according to the present

invention

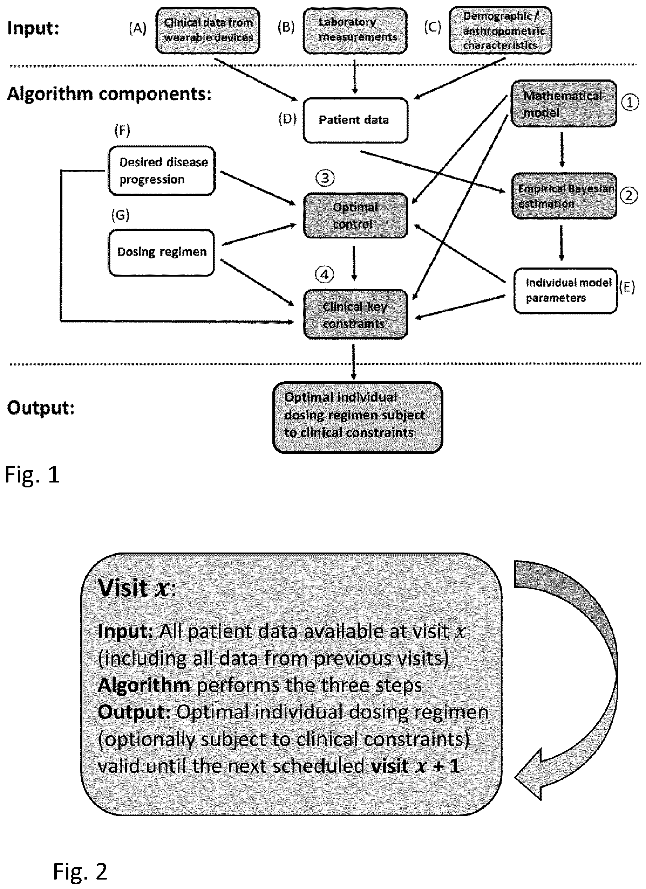

The algorithm particularly consists of four components (Figure 1; (1)-(4)) and their interaction. In addition, all additional components which interact with the components (1)-(4) are shown in Figure 1; (A)-(G). Explanation of all four components and their input/output relationships are presented below and in Figure 1.

Application of a mathematical model (Figure 1; (1))

The development of the mathematical model can be performed in two different ways. First, a mathematical model is already available. Then, this mathematical model can be applied in our algorithm and the step in this paragraph is skipped. Second, no such mathematical model is available and therefore, must be developed as follows:

Input:

> 1) Basic mathematical model
> 2) Non-linear mixed effects modeling approach
> 3) Retrospective patient data from a population

Output:

> 1) Mathematical model

Application of the empirical Bayesian estimation method (Figure 1: (2))

The empirical Bayesian estimation method estimates the individual model parameters for a new patient based on the mathematical model and their individual patient data. Input:

> 1) Mathematical model
> 2) Data of new patient

Output:

> 1) Individual model parameters of the new patient

Application of the optimal control algorithm (Figure 1; (3))

The optimal control problem is solved in particular with the OptiDose algorithm that computes the optimal dosing regimen for an individual patient.

Input:

> 1) Mathematical model

2) Individual model parameters of the new patient
3) Desired disease progression provided by a mathematical function
4) Dosing regimen

Output:

1) Optimal individual dosing regimen

Optionally, particularly if necessary: Adjustment of the optimal individual dosing regimen subject to clinical constraints (Figure 1; (4))
In an a-posteriori step, the optimal individual dosing regimen is adjusted to account for constraints in clinical application, e.g., limitations due to available tablet sizes.
Input:

1) Mathematical model
2) Individual model parameters of the new patient
3) Desired disease progression provided by a mathematical function
4) Clinical constraints
5) Optimal individual dosing regimen

Output:

1) Optimal individual dosing regimen subject to constraints in clinical application Particularly, the present invention combines a mathematical model, the empirical Bayesian estimation method, an optimal control algorithm and optionally the inclusion of constraints in clinical application in order to calculate an optimal individual dosing regimen for a patient subject to said constraints.

The method / algorithm according to the present invention is an iterative procedure over the number of visits (Figure 2). At every visit, the optimal individual dosing regimen, optionally subject to clinical constraints, is computed by performing one iteration of the algorithm. This optimal individual dosing regimen is valid until the next scheduled visit.
Performing one iteration of the algorithm at a visit:

Step 1) "Estimation of the individual model parameters": Based on the available patient data at the current and the past visits, and the administered doses until the current visit (Figure 1; (A)-(D)) the individual model parameters $\phi_i$ of the patient (Figure 1; (E)) are estimated with the empirical Bayesian estimation method utilizing the mathematical model (Figure 1; (1)-(2)).

Step 2) "Optimal individual dosing regimen": Estimated individual model parameters (Figure 1; (E)) are applied to compute the optimal individual dosing regimen (Figure 1; (1), (3), (F), (G)) for the time period until the next scheduled visit.

Step 3) (optional) "Optimal individual dosing regimen subject to clinical constraints ": The optimal individual dosing regimen (Figure 1; (3)) is adjusted to account for constraints in clinical application (Figure 1; (4), (E), (F), (G)).

These steps are iteratively repeated at every visit. At the first visit in this iterative process, only information at this visit is applied.
In a preferred embodiment of the present invention, the individual aspects of the present invention (particularly method, system, and computer-program according to the present invention) are applied to thyroid diseases in children and adults.
As already indicated above, thyroid hormones are essential for normal brain development, growth and puberty. However, treatment of the rare thyroid diseases congenital hypothyroidism (no or too low thyroid hormones production) and pediatric-onset Graves' disease (too high thyroid hormone production) is difficult. First, a carefully selected starting dose based on clinical experience is necessary. Second, after reaching a physiological balance of thyroid hormones, continuous adjustment of the individual dose depending on age, weight and disease severity is required to maintain euthyroidism. To mitigate the risk of negative neurological outcome, it is important to establish an optimal, individual dosing strategy that is continuously fine-tuned to account for specific needs in neonates, infants and children with hyper- or hypothyroidism.
Particularly, in an embodiment of the invention, the disease for which an optimal dosing regimen is to be determined, is one of the following four thyroid diseases which delivers a solution to the current medical problems formulated in the introduction.

According to an embodiment, as described above, an artificial neural network (ANN) can be included in the approach in order to calculate the optimal individual dosing regimen within a short amount of time, particularly within seconds. In other words, ANNs can be used to speed up the method, system and/or computer program/algorithms according to the present invention, but are however not necessary for the invention.

Patients with congenital hypothyroidism: For congenital hypothyroidism, an artificially manufactured T4 hormone (which is manufactured by different pharmaceutical companies in different galenic forms (e.g., tablets, drops), e.g., levothyroxine) is administered as substitution therapy to normalize the thyroid hormones (T3, FT3, T4, FT4, TSH) to the normal reference range. The overall concept from the main claim is applied to this disease area for pediatric patients starting at birth. A mathematical model for congenital hypothyroidism with levothyroxine substitution therapy was published by us in 2021 [Koch 2021].

Patients with acquired hypothyroidism: In analogy to congenital hypothyroidism, acquired hypothyroidism in children, pregnant women and adults can be modeled utilizing the same approaches.

Patients with acquired hyperthyroidism: For acquired hyperthyroidism, in pediatric and adult patients, two different treatment strategies are applied in clinical practice to normalize the thyroid hormones (T3, FT3, T4, FT4, TSH) to the normal reference range:

> 1) Administration of an anti-thyroid agent (e.g., carbimazole) alone to inhibit thyroid hormone overproduction
> 2) Simultaneous administration of an anti-thyroid agent (e.g., carbimazole) to inhibit thyroid function, and levothyroxine as substitution therapy to increase T4 hormones.

So far, dosing of carbimazole with or without simultaneous levothyroxine is based on laboratory testing of the thyroid hormones T3, T4, FT3, FT4, and TSH. A new approach will be the use of heart rate of the patient, a well-established very sensitive clinical sign for too high levels of thyroid hormones. Patients at diagnosis of hyperthyroidism have always too high heart rate values, which only normalize over weeks, when the thyroid hormone levels return to the normal range under carbimazole treatment. Heart rate will be monitored utilizing a wearable device. This allows to establish a relationship between FT4 and heart rate with the goal to provide medical advice and drug dosing at distance (telemedicine) based on resting heart rate without the need of clinical consultations and blood testing. This approach will be of importance especially at long distances and in low- and middle-income countries where laboratory testing is not everywhere available.

Patients with congenital hyperthyroidism: In analogy to acquired hyperthyroidism, congenital hyperthyroidism in pediatrics can be modeled by the same approaches.

Example of a mathematical model for congenital hypothyroidism

A mathematical model for levothyroxine treatment and the resulting FT4 concentration is presented [Koch 2021]. Due to the substitution therapy, this model can be considered as a pure PK model. A one-compartment model with absorption compartment and an additional endogenous production term was developed:

$$\frac{d}{dt}A_b^{T4}(t) = In^{T4}\big(t_k^{T4}, d_k^{T4}, F^{T4}\big) - k_a^{T4} \cdot A_b^{T4}(t) \qquad\qquad A_b^{T4}(0) = 0$$

$$\frac{d}{dt}T4(t) = k_a^{T4} \cdot A_b^{T4}(t) + k_{endo}^{T4} - k_{el}^{T4} \cdot T4(t) \qquad\qquad T4(0) = \frac{k_{endo}^{T4}}{k_{el}^{T4}}$$

$$C^{FT4}(t) = \frac{0.3 \cdot T4(t)}{V^{T4}(W)} \quad \text{with} \quad V^{T4}(W) = f_V^{T4} \cdot \left(\frac{W(t)}{W_{Ref}}\right)^{\beta_W}$$

where $C^{FT4}$ is the FT4 concentration resulting from levothyroxine treatment, $W$ the body weight (time-varying covariate) and $W_{Ref}$ a reference body weight (usually the average of body weight in the underlying patient population). The input function describing the dose administration is $In^{T4}$ where $t_k^{T4}$ is the $k$-th dosing time point, $d_k^{T4}$ the $k$-th dose of

levothyroxine and $F^{T4}$ a multiplicative factor, e.g., describing bioavailability of levothyroxine. The absorption compartment $A_b^{T4}$ characterizes, e.g., an oral administration, and compartment $T4$ characterizes the hormone thyroxine. The absorption rate is $k_a^{T4}$, the endogenous production rate is $k_{endo}^{T4}$ and the elimination rate is $k_{el}^{T4}$. Parameter $f_V^{T4}$ is a factor relating body weight with volume of distribution $V^{T4}$ and $\beta_W$ is a shape parameter. The vector combining all model parameters reads

$$\theta = \left(k_a^{T4}, k_{endo}^{T4}, k_{el}^{T4}, f_V^{T4}, \beta_W, F^{T4}\right)$$

Estimated individual model parameters $\psi_i = (k_{endo,i}^{T4}, f_{V,i}^{T4}, \beta_{W,i})$ were log-normally distributed.

Example of a mathematical model for acquired hyperthyroidism

A mathematical model for carbimazole treatment, additional block-and-replace (combination with levothyroxine) therapy, and the resulting free-thyroxine (FT4) concentration is presented. Structurally, this can be considered as a PKPD model where the PK is the methimazole (metabolized carbimazole) concentration caused from the carbimazole treatment and the PD is the FT4 biomarker. The developed model can be applied to both, carbimazole mono-therapy and block-and-replace therapy.

Carbimazole is a pro-drug and several properties of the PK, such as distribution, metabolism etc., are known. A detailed carbimazole PK model is presented. Let $d_l^C$ be the dose of carbimazole at time point $t_l^C$. Carbimazole absorption is characterized by

$$\frac{d}{dt}A_b^C(t) = In^C\!\left(t_l^C, d_l^C\right) - k_a^C \cdot A_b^C(t) \qquad\qquad A_b^C(0) = 0$$

where $k_a^C$ is the absorption rate and $In^C$ is the dosing input function of carbimazole. Amount of carbimazole $A^C$ in the central compartment is described by

$$\frac{d}{dt}A^C(t) = k_a^C \cdot A_b^C(t) - k_t \cdot A^C(t) \qquad\qquad A^C(0) = 0$$

where $k_t$ is the metabolism transit rate. Amount of methimazole $A^M$ in the central compartment is given by

$$\frac{d}{dt}A^M(t) = f^M \cdot k_t \cdot A^C(t) - k_{el}^M \cdot A^M(t) - k_{12} \cdot A^M(t) + k_{21} \cdot P(t) \qquad A^M(0) = 0$$

$$\frac{d}{dt}P(t) = k_{12} \cdot A^M(t) - k_{21} \cdot P(t) \qquad\qquad P(0) = 0$$

where $f^M$ is the metabolism conversion factor, $k_{el}^M$ is the elimination rate, and $k_{12}$ as well as $k_{21}$ are the distribution rates for the peripheral compartment $P$. Concentration of methimazole is obtained by

$$C^M(t) = \frac{A^M(t)}{V^M(W)} \quad \text{with} \quad V^M(W) = f_V^M \cdot W(t)$$

where $W(t)$ is the body weight over time and $f_V^M$ is a multiplicative factor linearly relating body weight with volume of distribution of methimazole $V^M$.

The inhibitory carbimazole treatment effect on the endogenous T4 production rate via the methimazole concentration $C^M(t)$ is included utilizing an inhibitory effect term

$$\frac{d}{dt}A_b^{T4}(t) = In^{T4}\left(t_k^{T4},d_k^{T4},F^{T4}\right) - k_a^{T4} \cdot A_b^{T4}(t) \qquad\qquad A_b^{T4}(0) = 0$$

$$\frac{d}{dt}T4(t) \qquad\qquad\qquad T4(0) = \frac{k_{endo}^{T4}}{k_{el}^{T4}}$$

$$= k_a^{T4} \cdot A_b^{T4}(t) + k_{endo}^{T4} \cdot \left(1 - \frac{I_{max} \cdot C^M(t)}{IC_{50} + C^M(t)}\right) - k_{el}^{T4} \cdot T4(t)$$

$$C^{FT4}(t) = \frac{0.3 \cdot T4(t)}{V^{T4}(W)}$$

with

$$V^{T4}(W) = f_V^{T4} \cdot \left(\frac{W(t)}{W_{Ref}}\right)^{\beta_W}$$

where $I_{max} \leq 1$ is the maximal inhibitory drug effect and $IC_{50}$ is the methimazole concentration causing the half-maximal inhibitory effect. In addition, $In^{T4}$ is the dosing input function, $d_k^{T4}$ the dose of levothyroxine at time point $t_k^{T4}$, $F^{T4} \leq 1$ the bioavailability of levothyroxine and $k_a^{T4}$ the respective absorption rate. Parameter $f_V^{T4}$ is a factor relating body weight with volume of distribution $V^{T4}$ and $\beta_W$ is a shape parameter. $C^{FT4}$ is the FT4 concentration resulting from carbimazole mono-therapy ($In^{T4}$ = 0) or block-and-replace therapy. The vector combining all model parameters readsuitl

$$\theta = \left(k_a^C, k_t, k_{el}^M, k_{12}, k_{21}, f_V^M, k_a^{T4}, k_{endo}^{T4}, I_{max}, IC_{50}, k_{el}^{T4}, f_V^{T4}, \beta_W, F^{T4}\right)$$

Estimated individual model parameters $\psi_i = (k_{endo,i}^{T4}, IC_{50,i}, f_{V,i}^{T4}, \beta_{W,i})$ were log-normally distributed. The covariates age at baseline (AGE: time-invariant continuous) and disease severity at baseline (categorical) impact the individual endogenous T4 production rate $k_{endo,i}^{T4}$:

$$\log\left(k_{endo,i}^{T4}\right) = \log\left(k_{endo,pop}^{T4}\right) + \beta_{k_{endo}^{T4}}^{AGE} \cdot AGE + \begin{cases} 0 & \text{for severe disease} \\ \beta_{k_{endo}^{T4}}^{Cat\,2} & \text{for moderate disease} \\ \beta_{k_{endo}^{T4}}^{Cat\,3} & \text{for mild disease} \end{cases}$$

The continuous and time-dependent weight $W(t)$ can be modeled by, e.g., a weight progression model given by a mathematical function, e.g., the Leffler function for neonates and infants, depending on additional model parameters which can be estimated utilizing weight measurements, or as a time-varying covariate with an interpolation between weight measurements.

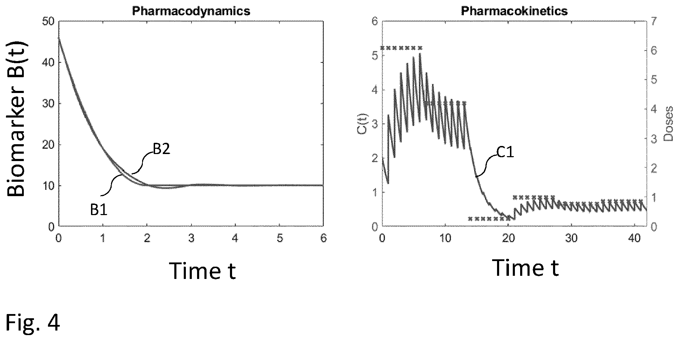

Finally, Fig. 4 depicts a schematic example of the optimal individual dosing regimen computed by the method/computer program according to the present invention.

Desired disease progression (left panel, solid curve B1) and the resulting biomarker concentration (left panel, solid curve B2) is shown for the computed optimal individual dosing regimen (right panel, crosses). The solid line C1 is the corresponding drug concentration resulting from the optimal individual dosing regimen.

**References**

[Polak 2017]: Polak M, Refetoff, S, Szinnai G, van Vliet G. Part III: Thyroid Gland Disorders. Pediatric Endocrinology and Inborn Errors of Metabolism. Editors: K. Sarafoglou, G.F. Hoffmann, K.S. Roth, McGraw-Hill Education, New York, 2nd Edition, 2017, pp. 481-512

[Leger 2014]: Léger J, Kaguelidou F, Alberti C, Carel JC. Graves' disease in children. Best Pract Res Clin Endocrinol Metab. 2014;28(2):233-243

[Belotti 2013]: Belotti P et al. (2013) Mixed-integer nonlinear optimization. Acta Numerica 22:1-131

[Lavielle 2014]: Lavielle M (2014) Mixed effects models for the population approach: models, tasks, methods and tools. Chapman & Hall / CRC Biostatistics Series Book 66

[Bassett 2018]: Bassett R, Deride J (2019) Maximum a posteriori estimators as a limit of Bayes estimators. Mathematical Programming 174:129-144

[Bachmann 2021]: Bachmann F, Koch G, Pfister M, Szinnai G, Schropp J (2021) OptiDose: computing the individualized optimal drug dosing regimen using optimal control. Journal of Optimization Theory and Applications 189:46-65

[Koch 2021]: Koch G et al. (2021) Modeling of levothyroxine in newborns and infants with congenital hypothyroidism: challenges and opportunities of a rare disease multi-center study. J Pharmacokinet Pharmacodyn 48(5):711-723

**Claims**

1. Method for determining an optimal individual dosing regimen of at least one drug for a patient suffering from a known disease, wherein the method comprises the steps of:

   (a) Providing a mathematical model adapted to model a disease progression of said disease and an effect of the at least one drug on the progression of the disease, the model comprising individual model parameters associated with the patient,
   (b) Utilizing an empirical Bayesian estimation to estimate the individual model parameters of the mathematical model utilizing patient data,
   (c) Calculating an optimal individual dosing regimen for the mathematical model by solving an optimal control problem based on a desired disease progression, the estimated individual model parameters, and an initial guess for the dosing regimen,

2. The method according to claim 1, wherein the method further comprises the step of:
   (d) Adjusting the optimal individual dosing regimen to account for at least one clinical constraint to yield the optimal individual dosing regimen subject to said at least one clinical constraint.

3. The method according to claim 1 or 2, wherein the mathematical model is a pharmacokinetic-pharmacodynamic (PKPD) model.

4. The method according to one of the preceding claims, wherein the desired disease progression is given in form of a mathematical function.

5. The method according to one of the preceding claims, wherein steps (b) to (c), particularly steps (b) to (d), are conducted at each visit of a plurality of *x* succeeding visits of the patient, wherein for *x* > 1 the patient data in step b) includes all patient data of the previous *x* - 1 visit(s) and the empirical Bayesian estimation in step b) further uses all doses of said at least one drug actually administered to the patient until the current visit.

6. The method according to one of the preceding claims, wherein at least a part of an algorithm performing steps (b) to (c), particularly steps (b) to (d), is approximated by an artificial neural network (ANN).

7. The method according to one of the preceding claims, wherein the disease is one of: acquired hypothyroidism, particularly autoimmune and/or non-autoimmune forms; acquired hyperthyroidism, particularly autoimmune and/or non-autoimmune forms; congenital hypothyroidism, congenital hyperthyroidism.

8. The method according to one of the preceding claims, wherein the drug is selected from the group comprised of: levothyroxine, carbimazole, propylthiouracil.

9. The method according to one of the preceding claims, wherein at least a portion of said patient data is measured by a wearable device worn by the patient.

10. The method according to claim 9, wherein the wearable device is one of: a watch, particularly smart watch; a mobile phone, particularly smart phone.

11. The method according to claim 9 or 10, wherein said patient data comprises the heart rate, wherein the heart rate of the patient is measured with the wearable device.

12. A system for determining an optimal individual dosing regimen of at least one drug for a patient suffering from a known disease, the system comprising at least one processor configured to perform the steps of the method according to one of the preceding claims.

13. The system according to claim 12, wherein the system further comprises a wearable device configured to measure at least a portion of said patient data.

14. A computer program comprising instructions, which when carried out on the system according to claim 12 or 13, cause the system to carry out the method according to one of the claims 1 to 11.

15. A computer-readable non-transitory storage medium having stored thereon the computer program according to claim 14.

16. A data carrier signal carrying the computer program of claim 14.

**Input:**

(A) Clinical data from wearable devices (B) Laboratory measurements (C) Demographic / anthropometric characteristics

**Algorithm components:**

(D) Patient data

Mathematical model ①

(F) Desired disease progression

③ Optimal control

Empirical Bayesian estimation ②

(G) Dosing regimen

④ Clinical key constraints

Individual model parameters (E)

**Output:**

Optimal individual dosing regimen subject to clinical constraints

Fig. 1

**Visit $x$:**

**Input:** All patient data available at visit $x$ (including all data from previous visits)
**Algorithm** performs the three steps
**Output:** Optimal individual dosing regimen (optionally subject to clinical constraints) valid until the next scheduled **visit $x + 1$**

Fig. 2

Fig. 3

Fig. 4

Europäisches
Patentamt
European
Patent Office
Office européen
des brevets

## EUROPEAN SEARCH REPORT

**Application Number**

EP 21 20 6570

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/273578 A1 (KUTZKO JOHN D [US] ET AL) 27 August 2020 (2020-08-27) * paragraph [0092] – paragraph [0186] * ----- | 1–16 | INV. G16H20/10 G16H50/50 |
| A | US 2020/350073 A1 (GOLDSMITH PAUL [GB] ET AL) 5 November 2020 (2020-11-05) * paragraph [0041] – paragraph [0195] * ----- | 1–16 | |

TECHNICAL FIELDS
SEARCHED      (IPC)

G16H

The present search report has been drawn up for all claims

| Place of search | Date of completion of the search | Examiner |
|---|---|---|
| Munich | 23 March 2022 | Rinelli, Pietro |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.......................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 20 6570

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2022

| Patent document cited in search report | | Publication date | Patent family member(s) | | Publication date |
|---|---|---|---|---|---|
| US 2020273578 | A1 | 27-08-2020 | US 2020273578 | A1 | 27-08-2020 |
| | | | US 2021241918 | A1 | 05-08-2021 |
| US 2020350073 | A1 | 05-11-2020 | AU 2020264769 | A1 | 23-12-2021 |
| | | | BR 112021021976 | A2 | 11-01-2022 |
| | | | CA 3138387 | A1 | 05-11-2020 |
| | | | CN 113906518 | A | 07-01-2022 |
| | | | EP 3963602 | A1 | 09-03-2022 |
| | | | KR 20220005073 | A | 12-01-2022 |
| | | | SG 11202111929T | A | 29-11-2021 |
| | | | US 2020350073 | A1 | 05-11-2020 |
| | | | WO 2020221993 | A1 | 05-11-2020 |

EPO FORM P0459

For more details about this annex : see Official Journal of the European Patent Office, No. 12/82

**EP 4 177 902 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BACHMANN F ; KOCH G ; PFISTER M ; SZINNAI G ; SCHROPP J.** OptiDose: Computing the Individualized Optimal Drug Dosing Regimen Using Optimal Control. *Journal of Optimization Theory and Applications,* 2021, vol. 189, 46-65
- *CHEMICAL ABSTRACTS,* 51-48-9
- *CHEMICAL ABSTRACTS,* 22232-54-8
- *CHEMICAL ABSTRACTS,* 60-56-0
- *CHEMICAL ABSTRACTS,* 51-52-5
- Part III: Thyroid Gland Disorders. **POLAK M ; REFETOFF, S ; SZINNAI G ; VAN VLIET G.** Pediatric Endocrinology and Inborn Errors of Metabolism. McGraw-Hill Education, 2017, 481-512
- **LÉGER J ; KAGUELIDOU F ; ALBERTI C ; CAREL JC.** Graves' disease in children. *Best Pract Res Clin Endocrinol Metab.,* 2014, vol. 28 (2), 233-243
- **BELOTTI P et al.** Mixed-integer nonlinear optimization. *Acta Numerica,* 2013, vol. 22, 1-131
- **LAVIELLE M.** Mixed effects models for the population approach: models, tasks, methods and tools. Chapman & Hall / CRC Biostatistics, 2014, vol. 66
- **BASSETT R ; DERIDE J.** Maximum a posteriori estimators as a limit of Bayes estimators. *Mathematical Programming,* 2019, vol. 174, 129-144
- **BACHMANN F ; KOCH G ; PFISTER M ; SZINNAI G ; SCHROPP J.** OptiDose: computing the individualized optimal drug dosing regimen using optimal control. *Journal of Optimization Theory and Applications,* 2021, vol. 189, 46-65
- **KOCH G et al.** Modeling of levothyroxine in newborns and infants with congenital hypothyroidism: challenges and opportunities of a rare disease multi-center study. *J Pharmacokinet Pharmacodyn,* 2021, vol. 48 (5), 711-723